(19) Europäisches Patentamt
European Patent Office
Office européen des brevets

(11) EP 2 564 772 A1

(12) EUROPEAN PATENT APPLICATION
published in accordance with Art. 153(4) EPC

(43) Date of publication:
06.03.2013 Bulletin 2013/10

(51) Int Cl.:
*A61B 5/053* (2006.01)

(21) Application number: 10850777.3

(22) Date of filing: 28.04.2010

(86) International application number:
PCT/KR2010/002696

(87) International publication number:
WO 2011/136409 (03.11.2011 Gazette 2011/44)

(84) Designated Contracting States:
AT BE BG CH CY CZ DE DK EE ES FI FR GB GR
HR HU IE IS IT LI LT LU LV MC MK MT NL NO PL
PT RO SE SI SK SM TR

(71) Applicant: M.I. Tech Co., Ltd.
Gyeonggi-do 451-864 (KR)

(72) Inventors:
• PARK, Se-Ik
Pyeongtaek-si, Gyeonggi-do 451-864 (KR)

• LEE, Hoseung
Pyeongtaek-si, Gyeonggi-do 451-864 (KR)

(74) Representative: Kindermann, Peter
Kindermann Patentanwälte
Postfach 10 02 34
85593 Baldham (DE)

(54) APPARATUS FOR MEASURING INTERFACIAL IMPEDANCE BETWEEN THE BODY AND A STIMULATING ELECTRODE

(57) The present invention relates to an apparatus for measuring interfacial impedance between the body and a stimulating electrode, comprising: a first electrode connected to some cells in the body; a second electrode connected to other cells in the body so as to provide a current which is applied by a stimulator to the first electrode through the cells; a measurement unit for selectively extracting voltages loaded on the first and second electrodes according to currents applied to the first and second electrodes; a charge storage unit to which a relative potential corresponding to a voltage difference between the first and second electrodes is stored; an A/D conversion unit for converting the signal corresponding to the relative potential into a digital signal and for outputting the digital signal; and an impedance calculation unit for calculating the interfacial impedance between the first and second electrodes according to the digital signal outputted from the A/D conversion unit and the current applied to the second electrode. Therefore, according to the present invention, interfacial impedance between the body and a stimulating electrode implanted to the brain or muscle of the body can be measured by using the difference in voltages loaded on each electrode.

FIG.1

## Description

### TECHNICAL FIELD

The present invention relates to an apparatus for analyzing electrochemical characteristics of a stimulating electrode that handles a function of transferring an electric stimulating signal generated from a nerve stimulator, which electrically stimulates a nerve or a muscle to recover a lost sensitive facility or motor function, to a nerve or a muscle in a contact manner; and more particularly, to an apparatus for measuring interfacial impedance between a living body and a stimulating electrode, which is capable of accurately measuring the change of interfacial impedance between the body and the stimulating electrode depending on the lapse of time and a bio-environment around the electrode in real time after the stimulating electrode is transplanted in the living body.

### BACKGROUND OF THE INVENTION

Various sense and motor functions of a human body are controlled or affected by a nervous activity of a brain. A nervous activity of a brain has inhibition or excitability, and appropriate balancing of these two functions allows appropriateness of sense and motor functions to be maintained. However, damage to the balancing of such functions due to injury of a brain, illness, disease, and the like may cause disorder of pathological neurility. For example, stroke caused by blockage or bursting of a blood vessel in the brain may considerably degrade excitability of a cerebral nerve function to bring about weakening of motor, disorder of sense, a speech disorder, a perceptual disorder, and the like. Conversely, epilepsy excessively increases excitability of a particular portion of a brain to induce abnormal excessive nervous activity, which causes a physical seizure. And, degenerative brain diseases such as Parkinson's disease or dementia show disease symptoms such as tremor (essential tremor), bradykinesia, amnesia, or the like. Also, damage to the peripheral nervous system or the central nervous system due to disease, accidents or the like may induce severe disabilities such as hearing impairment, visual impairment, local or general paralysis, and the like.

In the case of the symptoms resulting from the foregoing causes, a lost sensory function or motor function may be recovered by applying appropriate electrostimulation to the central nervous system or the peripheral nervous system, and typical electrostimulation includes a cochlear implantation that recovers auditory sense or a deep brain stimulator for recovering a motor function lost due to Parkinson's disease. On the strength of a success of such devices in the market, recently, a treatment of an illness by applying electrostimulation to an epileptic, a patient suffering from Alzheimer's, a melancholiac, a patient who is obsessive-compulsive has been actively researched and attempted.

In order to transfer a signal generated from a stimulator generating electrostimulation inducing a treatment effect to patients to the central nervous system or the peripheral nervous system, electrodes having various forms and structures are required. In general, such electrodes are completely transplanted to a living body, and interfacial impedance, which is electrochemical characteristics of the transplanted electrodes are changed over time due to a humoral immune reaction, an electrostimulation condition, or the like. Such a change in impedance changes an amount of charge transmission in transmitting an electrical signal to a living body and also affects a setting of the stimulator generating the electrical signal. Thus, in order to stably generate a continuous electrical stimulating signal inducing an effective treatment effect to patients, it is very important to quickly and accurately measure interfacial impedance of an electrode transplanted in individual patients in real time. One of example of an impedance measurement apparatus may include U.S. Patent No. 4,870,341, entitled "IMPEDANCE MEASUREMENT CIRCUIT" and issued to James M. Pihl et al., on September 26, 1989.

In the state of the art for measuring interfacial impedance, an AC voltage having a small magnitude that does not induce an oxidation-reduction reaction on a surface of an electrode is applied to an electrode at a different frequency, and then a corresponding AC current is measured to measure interfacial impedance. Or, a short pulse is repeatedly applied to an electrode, based on which interfacial impedance is inferred. However, these methods utilize a D/A converter, an A/D converter, an oscillator, and the like with high precision and a complicated structure, causing much power consumption and having a long measurement time. In addition, since such a structure requires a number of components, when a system semiconductor chip, as a core component of a small electric nerve stimulator transplanted in a living body, is fabricated, costs are increased due to an increase in the area. Also, a great amount of power consumption shortens a lifetime of the nerve stimulator.

### DISCLOSURE

### TECHNICAL PROBLEM

In view of the above, therefore, the present invention provides an apparatus for accurately measuring the change in impedance of an electrode transplanted in a living body in real time through a simple configuration.

TECHNICAL SOLUTION

In accordance with an aspect of the present invention, there is provided an apparatus for measuring interfacial impedance between a living body and a stimulating electrode, the apparatus including: a first electrode connected to one end of a cell within the living body; a second electrode connected to the other end of the cell within the living body, wherein a current is applied from a stimulator and is provided to the first electrode through the cell; a measurement unit configured to selectively extract a voltage induced to the first and second electrodes depending on the current applied to the first and second electrodes; a charge storage unit configured to store a relative potential corresponding to a voltage difference between the first and second electrodes; an A/D conversion unit configured to convert a signal corresponding to the relative potential into a digital signal; and an impedance calculation unit configured to calculate interfacial impedance of the first and second electrodes using the digital signal from the A/D conversion unit and the current applied to the second electrode.

In an exemplary embodiment of the apparatus, the measurement unit includes: a first switching unit connected to the first electrode and switched to connect the first electrode to the charge storage unit so that a voltage at the first electrode is stored in the charge storage unit; and a second switching unit connected to the second electrode and switched to connect the second electrode to the charge storage unit so that a voltage at the second electrode is stored in the charge storage unit, wherein when the first switching unit is switched on or off, the second switching unit is switched off or on.

In an exemplary embodiment of the apparatus, the apparatus further includes: a third switching unit connected in parallel to the charge storage unit to perform a switching operation; a fourth switching unit connected in parallel to the charge storage unit to perform a switching operation; and a voltage follower connected in parallel to the fourth switching unit, wherein the voltage follower provides an output signal corresponding to the relative potential stored in the charge storage unit to the A/D conversion unit as the relative potential is applied to one end thereof, and receives feedback of the output signal by the other end thereof.

In an exemplary embodiment of the apparatus, the voltage at the first electrode is stored in the charge storage unit when the first and fourth switching units are turned on and the second and third switching units are turned off, and the relative potential between the first and second electrodes is applied to one end of the voltage follower when the second switching unit is turned on and the first, third, and fourth switching units are turned off.

In an exemplary embodiment of the apparatus, a charge corresponding to the relative potential stored in the charge storage unit is discharged when the first and second switching units are turned off and the third and fourth switching units are turned on.

In an exemplary embodiment of the apparatus, the measurement unit includes: first and second resistors R1 and R2 connected in parallel to the first electrode; third and fourth resistors R3 and R4 connected in parallel to the second electrode; a fifth switching unit connected between the first and second resistors R1 and R2 to connect the first electrode to the charge storage unit through a switching operation; a sixth switching unit connected in series to the second resistor R2 to earth the second resistor R2 through a switching operation; a seventh switching unit connected between the third and fourth resistors R3 and R4 to connect the second electrode to the charge storage unit through a switching operation; and an eighth switching unit connected in series to the fourth resistor R4 to earth the fourth resistor R4 through a switching operation.

In an exemplary embodiment of the apparatus, the fifth and sixth switching units are simultaneously switched on or off, and the seventh and eighth switching units are simultaneously switched on or off.

In an exemplary embodiment of the apparatus, the apparatus further includes: a third switching unit connected in parallel to the charge storage unit to perform an ON or OFF switching operation; a fourth switching unit connected in parallel to the charge storage unit to perform an ON or OFF switching operation; and a voltage follower connected in parallel to the fourth switching unit, wherein the voltage follower provides an output signal corresponding to the relative potential stored in the charge storage unit to the A/D conversion unit as the relative potential is applied to one end thereof, and receive feedback of the output signal by the other end thereof.

In an exemplary embodiment of the apparatus, the voltage at the first electrode is stored in the charge storage unit when the fourth, fifth, and sixth switching units are turned on and the third, seventh, and eighth switching units are turned off, and a relative potential between the first and second electrodes is applied to one end of the voltage follower when the seventh and eighth switching units are turned on and the third, fourth, fifth, and sixth switching units are turned off.

In an exemplary embodiment of the apparatus, a charge corresponding to the relative potential stored in the charge storage unit is discharged when the fifth, sixth, seventh, and eighth switching units are turned off and the third and fourth switching units are turned on.

In an exemplary embodiment of the apparatus, the impedance calculation unit calculates impedance by time at certain time intervals by using each digital signal from the A/D conversion unit and the current applied to the second electrode, and wherein the calculated impedance by time is assigned a unique index corresponding to measurement order.

In an exemplary embodiment of the apparatus, the impedance calculation unit restores a voltage waveform taken to both ends of the first and second electrodes by using the impedance by time and the unique index to calculate a time constant in the interfacial impedance of the first and second electrodes.

In an exemplary embodiment of the apparatus, the first and second electrodes are configured to be bipolar in which a pair of stimulating electrodes has the area with the same size.

In an exemplary embodiment of the apparatus, the impedance calculation unit calculates a half value of impedance calculated by using the current applied to the second electrode and a digital signal from the A/D conversion unit, and wherein the calculated half value becomes the interfacial impedance of the first and second electrodes.

In an exemplary embodiment of the apparatus, the first and second electrodes are configured to be monopolar in which the area of the second electrode is greater than that of the first electrode.

In an exemplary embodiment of the apparatus, the impedance calculation unit calculates impedance calculated by using the current applied to the second electrode and a digital signal output from the A/D conversion unit, and wherein the calculated impedance becomes the interfacial impedance of the second electrode.

## ADVANTAGEOUS EFFECTS

In accordance with the present invention, the apparatus for measuring impedance of each electrode uses a voltage difference induced to both ends of each electrode transplanted to a living body and a current stimulating signal is applied to the pair of electrodes is provided, thereby accurately measuring impedance of the electrodes changing depending on a transplantation period and an environment in real time through a simple configuration.

## BRIEF DESCRIPTION OF THE DRAWINGS

Fig. 1 illustrates a circuit diagram of an apparatus for measuring interfacial impedance of a stimulating electrode in accordance with an embodiment of the present invention;

Fig. 2 is a view illustrating a voltage waveform by an RC component and a process of storing digital values in accordance with the embodiment of the present invention;

Fig. 3 is an equivalent circuit diagram when an electrode is configured as a bipolar electrode in accordance with the embodiment of the present invention;

Fig. 4 is an equivalent circuit diagram when an electrode is configured as a monopolar electrode in accordance with the embodiment of the present invention; and

Fig. 5 illustrates a circuit diagram of another embodiment of the measurement unit in the apparatus for measuring impedance in accordance with an embodiment of the present invention.

## BEST MODE FOR THE INVENTION

The advantages and features of embodiments and technical constructions of accomplishing these will be clearly understood from the following embodiments taken in conjunction with the accompanying drawings. In the following description, well-known functions or constitutions will not be described in detail if they would obscure the invention in unnecessary detail. Further, the terminologies to be described below are defined in consideration of functions in the invention and may vary depending on a user's or operator's intention or practice.

The embodiments are not limited and may be implemented in various forms. It should be noted that the embodiments are provided to make a full disclosure and also to allow those skilled in the art to understand the full range of the embodiments. Therefore, embodiments are to be defined only by the scope of the appended claims.

A measurement apparatus for accurately measuring impedance of a stimulating electrode transplanted in a body part, e.g., a brain or a muscle, in accordance with an embodiment of the present invention will be described in detail with the accompanying drawings.

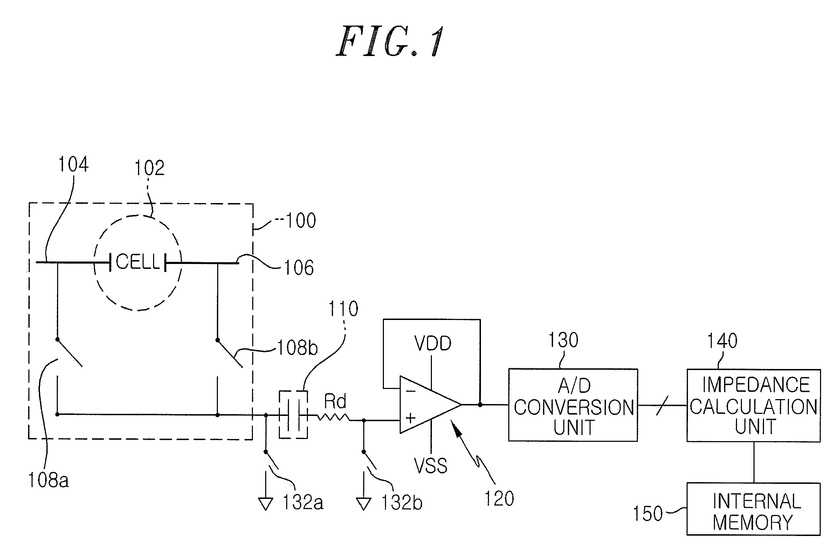

Fig. 1 illustrates a circuit diagram of an apparatus for measuring interfacial impedance of a stimulating electrode transplanted in a body part in accordance with an embodiment of the present invention. The apparatus includes a measurement unit 100; a charge storage unit 110; a voltage follower 120; an A/D conversion unit 130; first and second switching units 108a and 108b; third and fourth switching units 132a and 132b; an impedance calculation unit 140; and an internal memory 150.

As illustrated in Fig. 1, the measurement unit 100 includes first and second electrodes 104 and 106 connected to different ends of a bio-internal cell 102, the first switching unit 108a connected to the first electrode 104, and the second switching unit 108b connected to the second electrode 106.

Herein, the first and second electrodes 104 and 106 are stimulating electrodes transplanted in the bio-cell 102 of a body part to apply a current generated from a stimulator (not shown), through which a current may be applied to the bio-cell of the body part to stimulate the bio-cell. The first electrode 104 is transplanted in an end of the bio-cell 102 to allow a current to be applied to the second electrode 106 passing through the bio-cell 102, and the second electrode 106 is transplanted in the other end of the bio-cell 102 and a current generated from the stimulator is applied thereto. The first and second electrodes 104 and 106 may be configured to be bipolar in which a pair of stimulating electrodes have the area with the same size (the same impedance), or may be configured to be monopolar in which the area of the second electrode 106 is greater than that of the first electrode 104.

The first switching unit 108a is connected to the first electrode 104 to connect the first electrode 104 to the charge storing unit 110 through its ON switching operation, to thus provide a voltage from the first electrode 104 to the charge storage unit 110. In order to provide the voltage from the first electrode 104 to the charge storage unit 110, the fourth switching unit 132b, as well as the first switching unit 108a, needed to be turned on, and the second and third switching unit 108b and 132a needed to be turned off. The second switching unit 108b is connected to the second electrode 106 to connect the second electrode 106 to the charge storage unit 110 through its ON operation.

In the measurement unit 100, when the first switching unit 108a is turned on or off, the second switching unit 108b is turned off or turned on. In an operation of the measurement unit 100, the measurement unit 100 is configured to selectively provide a voltage depending on a current applied to the first electrode 104, and to this end, the first electrode 104 of the measurement unit 100 is connected to a body part, e.g., to the cell 102 of a brain or a portion of muscle. When the first switching unit 108a and the fourth switching unit 132b are in an ON state and the second and third switching units 108b and 132a are in an OFF state, an absolute potential E1 taken by a current flowing in the first electrode 104 is stored in the charge storage unit 110.

The second electrode 106 is connected to the other end of the cell 102 and has a potential E2 different from the absolute potential E1 applied to the first electrode 104 depending on interfacial impedance of the two electrodes connected to the cell 102. That is, as the first switching unit 108 is turned off, the second switching unit 108b connected to the second electrode 106 is turned on, and the third and fourth switching units 132a and 132b are turned off, input resistance of the voltage follower 120 has a significantly great value, and therefore, the absolute potential E1 as a charge stored in the charge storage unit 110 cannot be discharged. Thus, an input terminal potential of the voltage follower 120 maintains a value, which is obtained by subtracting the absolute potential E1 stored in the charge storage unit 110 from the potential E2 of the second electrode 106, that is, a relative potential E2-E1. The relative potential E2-E1 applied to the input terminal of the charge storage unit 110 is provided to the A/D conversion unit 130 through the voltage follower 120.

The third switching unit 132a is connected in parallel to the charge storage unit 110 and connected in series to the measurement unit 100 to perform an ON or OFF operation, and the fourth switching unit 132b is connected in parallel between the charge storage unit 110 and the voltage follower 120 to perform an ON or OFF operation.

The relative potential E2-E1 applied to the input terminal of the charge follower 120 is A/D-converted and is then discharged as the first switching unit 108a and the second switching unit 108b are turned off and the third switching unit 132a and the fourth switching unit 132b are turned on, so as to be turned to a standby state for extracting a next voltage.

In the embodiment of the present invention, the charge storage unit 110 serves to store a charge, which may be, for example, a capacitor. A damping resistor Rd is connected between the charge storage unit 110 and the voltage follower 120 in order to adjust a maximum value of a current abruptly flowing depending on the discharge of the relative potential E2-E1.

The voltage follower 120 has one end connected in parallel to the fourth switching unit 132b, and the relative potential stored in the charge storage unit 110 is applied to the one end thereof, whereby the voltage follower 120 provides a signal corresponding to the relative potential to the A/D conversion unit 130, and the other end to receives feedback of an output signal generated depending on the application of the relative potential. The voltage follower 120 may be an amplifier in which input impedance has a very great voltage relative to output impedance and of which a gain is "1" and maintains the stored charge of the charge storage unit 110 without discharging, thus serving as a buffer to generate a fixed input signal provided to the A/D conversion unit 130.

The A/D conversion unit 130 converts a signal output from the voltage follower 120 into a digital signal.

The impedance calculation unit 140 calculates interfacial impedance between the cell 102 and the first and second electrodes 104 and 106 by applying the output value, i.e., the relative potential, generated from the A/D conversion unit 130 and a current value supplied between the first and second electrodes 104 and 106 to Ohm's law. The calculated interfacial impedance is assigned a unique index which may be a measurement order by time and is then stored in the internal memory 150.

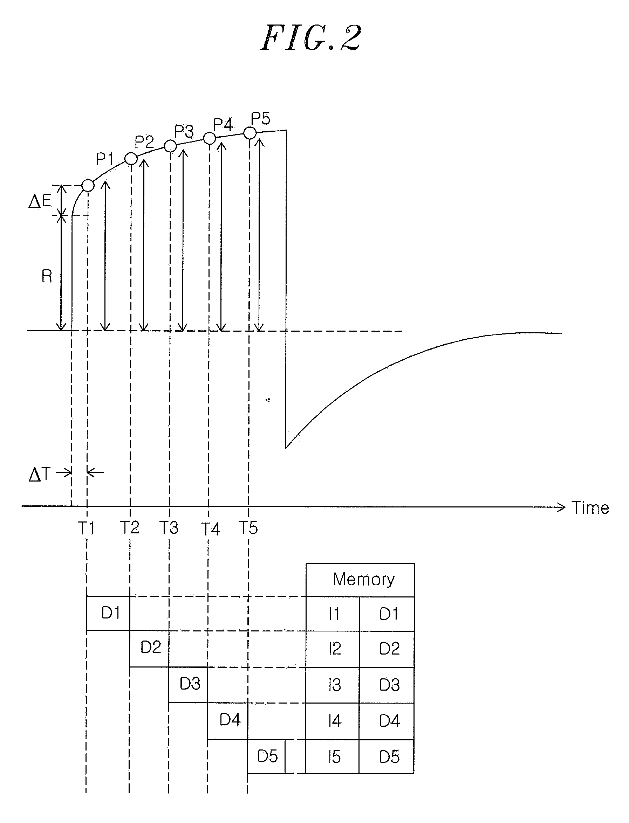

Meanwhile, the impedance calculation unit 140 may repeatedly perform the foregoing operation at certain time intervals. In other words, as illustrated in Fig. 2, the value of the potential difference P1, which is the relative potential E2-E1 measured at a time T1, is converted into a digital value D1 by the A/D conversion unit 130, and the impedance calculation unit 140 assigns an index L1 to the digital value D1 and stores the same in the internal memory 150. Herein, the index may refer to a measurement order. Digital values D2, D3, D4, and D5 for the potential differences P2, P3, P4, and P5 measured at the times T2, T3, T4, and T5 through the foregoing process are assigned different indices L2, L3, L4, and L5, and then stored in the internal memory 150. The impedance calculation unit 140 may analyze a measured voltage waveform and calculate impedance Z of the cell 102 and an RC time constant to calculate a resistance component R and a capacitor component C. In other words, the impedance calculation unit 140 may restore a voltage waveform by using the index indicating a measurement order, the measured digital value, and the number (i.e., the number of times of measurement) of the values stored in the internal memory 150, and may calculate the interfacial impedance Z between the cell 102 and the electrode based on the restored voltage waveform. In addition, as illustrated in Fig. 2, in order to accurately obtain the interfacial impedance Z between the cell 102 and the electrode, a voltage must be measured within a short time as possible after a current is applied to the electrode. That is, as $\Delta T$ is shorter, $\Delta E$ may be reduced and more accurate voltage waveform may be restored.

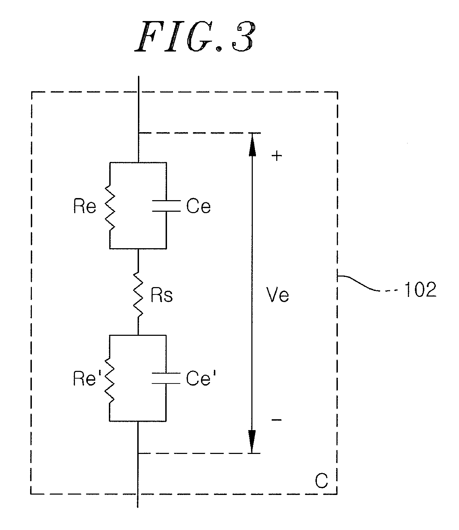

Meanwhile, the impedance Z calculated by the impedance calculation unit 140 includes resistance component values Rs, Re, and Re' as illustrated in Fig. 3. Fig. 3 shows an electrochemical equivalent circuit diagram of the cell represented by the cell 102 in Fig. 1 and the first and second electrodes 104 and 106 inserted into the cell, wherein the first and second electrodes 104 and 106 are configured as bipolar electrodes. That is, one of the first and second electrodes 1004 and 106 having the same area is used as a stimulating electrode and the other is used as a reference electrode, and therefore, it may be assumed that impedance of the stimulating electrode and that of the reference electrode are equal. Accordingly, it may be considered that a half value of the impedance Z calculated by the impedance calculation unit 140 is impedance of one electrode.

As mentioned above, the impedance calculation unit 140 restore a voltage waveform using the index indicating the number of times of measurement, the measured digital value, and the value stored in the internal memory 150 to calculate values Rs, Re, Ce, Re', and Ce'. That is, the impedance calculation unit 140 may calculate resistance components such as Rs, Re, and Re' and capacitor components such as Ce and Ce' from the impedance Z and the RC time constant.

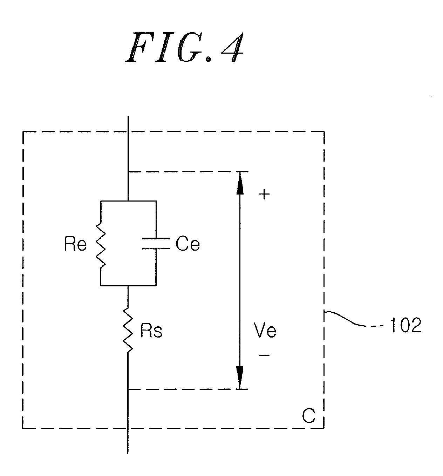

Fig. 4 is an equivalent circuit diagram when an electrode is configured as a monopolar electrode in accordance with the embodiment of the present invention. It is assumed that an interfacial impedance value of the first electrode 104 as a reference electrode is smaller by 10 times or more than that of the second electrode 106 as a stimulating electrode since the first electrode 104 has a surface area significantly greater than that of the second electrode 106. Then, measured impedance may be approximated only with the interfacial impedance of the first electrode 104 without a great error. Also, in the case of the monopolar electrode, an RC time constant may be calculated from the waveform restored through the impedance calculation unit 140, and a C value of the electrode may be inferred from the RC time constant.

Referring further to the interfacial impedance between the electrode and the living body, in Fig. 2, the value of the potential difference P1 corresponds to double R2 in case of the bipolar electrode, and corresponds to Re in case of the monopolar electrode, which are the resistance components illustrated in Figs. 3 and 4. The value of the potential difference P5 in Fig. 2 is approximate to the entire interfacial impedance Z between the living body and the electrode.

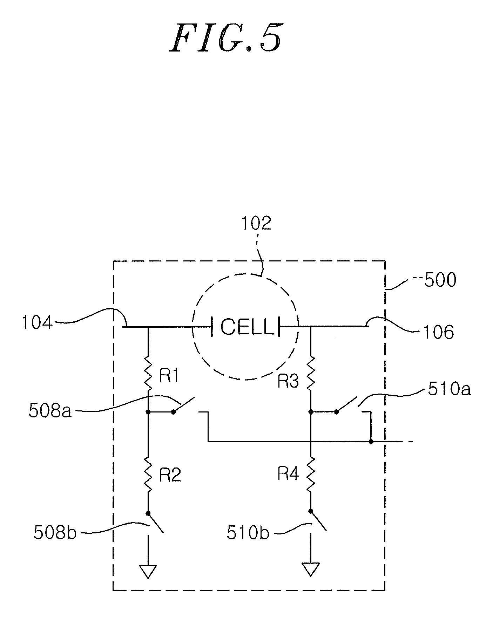

A nerve stimulator employing the impedance measurement apparatus in accordance with the embodiment of the present invention is a stimulator expressing a current as an output signal, and in general, up to a current magnitude of about 10 mA is applied to the cell 102 of a bio-cell. However, an average value of the impedance of the electrode equivalent circuits as illustrated in Figs. 3 and 4 is about 500 ohm to 1000 ohm, and thus, a voltage value as an absolute potential induced to the first and second electrodes 104 and 106 is as high as 5 to 10V. Therefore, in order to store such a high voltage in the capacitor as the charge storage unit 110, the voltage drop by the first and second switching units 108a and 108b should be nearly avoided. Thus, in the foregoing circuit configuration, elements used for the first and second switching units 108a and 108b, which derive a relative potential with an induced high level across the electrode, may require a component such as a level shifter, which generally operates at a voltage lower than 3.3V, to achieve a stable and safe switching. However, when a level shifter is employed in the circuit configuration, the circuit is complicated and power consumption is also increased. Thus, in accordance with another embodiment of the present invention, the impedance measurement apparatus is configured such that the switching elements connected to the first and second electrodes 104 and 106 are operated at a voltage lower than 3.3V, as illustrated in Fig. 5.

Fig. 5 illustrates a circuit diagram of an apparatus for measuring impedance in accordance with another embodiment of the present invention. The impedance apparatus in this embodiment has the same configuration and same function as the impedance measurement apparatus illustrated in Fig. 1, except for the measurement unit in the impedance measurement apparatus. Therefore, a detailed description thereof will be omitted and only the measurement unit will be described hereinafter.

Referring to Fig. 5, a measurement unit 100 in accordance with another embodiment of the present invention may include resistors R1 and R2 connected in parallel to the other end of the first electrode 104 having one end to which the cell 102 is connected, a fifth switching unit 508a connected in parallel to the resistor R1 for performing an ON/OFF operation to make a connection of a voltage induced to the first electrode 104 to the charge storage unit 110, a sixth switching unit 508b for performing an ON/OFF operation to make the connection of the resistor R2 to the earth, resistors R3 and R4 connected in parallel to the other end of the second electrode 106 having one end to which the cell 102 is connected, a seventh switching unit 510a connected in parallel to the resistor R3 for performing an ON/OFF operation to make the connection a voltage induced to the second electrode 106 to the charge storage unit 110, and an eighth switching unit 510b for performing an ON/OFF operation to make the connection of the resistor R4 to the earth. Herein, the fifth and seventh switching units 508a and 508b simultaneously perform ON/OFF operation, and the seventh and eighth switching units 510a and 510b also simultaneously perform ON/OFF operation. However, all of the fifth, sixth, seventh, and eighth switching units 508a, 508b, 510a, and 510b are not turned on simultaneously.

Following is a description on a process of applying a voltage by the measurement unit 100 having the foregoing configuration depending on an ON/OFF operation of the respective switching units. In operation, when the fourth, fifth, and sixth switching units 132b, 508a and 508b are turned and the third, seventh, and eighth switching units 132a, 510a, and 510b are turned off, an absolute potential E1 derived by a current flowing in the first electrode 104 is provided to and stored in the charge storage unit 110.

Meanwhile, when the seventh and eighth switching units 510a and 510b are turned on and the third, fourth, fifth, and sixth switching units 132a, 132b, 508a, and 508b are turned off, input resistance of the voltage follower 120 has a considerably great value, preventing the absolute potential E1 as a charge stored in the charge storage unit 110 from being discharged. Therefore, a potential at the input terminal of the voltage follower 120 is maintained as a value obtained by subtracting the absolute potential E1 stored in the charge storage unit 110 from the potential E2 of the second electrode 106, i.e., the relative potential E2-E1. The relative potential E2-E1 applied to the input terminal of the voltage storage unit 110 is provided to the A/D conversion unit 130 through the voltage follower 120.

The relative potential E2-E1 applied to the input terminal of the charge follower 120 is A/D-converted and is then discharged as the fifth, sixth, seventh, and eighth switching units 508a, 508b, 510a, and 510b are turned off and the third switching unit 132a and the fourth switching unit 132b are turned on, so as for the measurement unit to be turned to an idle state for extracting a next voltage.

Meanwhile, as mentioned above, a voltage value applied to drive the fifth, sixth, seventh, and eighth switching units 508a, 508b, 510a, and 510b may be adjusted to be lower than 3.3V by using the resistors R1 and R2 connected to both ends of the fifth and sixth switching units 508a and 508b and the resistors R3 and R4 connected to both ends of the seventh and eighth switching units 510a and 5 10b.

A voltage difference of an input to the charge storage unit 110 or the voltage follower 120 is expressed by Eq. (1) to Eq. (3) shown below.

$$V5 = K1 \times V1 \qquad \text{Eq. (1)}$$

$$V7 = K2 \times V2 \qquad \text{Eq. (2)}$$

$$V4 = V7 - V5 = \{(K2 - K1) \times V2\} + \{K1 \times (V2 - V1)\} \qquad \text{Eq. (3)}$$

In Eq. (1), V1 is a voltage induced to the first electrode 104 in Fig. 5, V5 is a voltage divided by the resistors R1 and R2, i.e., a voltage between a ground and the resistor R2, and K1 is R2/(R1+R2). Similarly, in Eq. (2), V2 is a voltage induced to the second electrode 106, V7 is a voltage divided by the resistors R3 and R4, i.e., a voltage between a ground and the resistor R4, and K2 is R4/(R3+R4). In Eq. (3), V4 is a voltage at a non-inverting input terminal of the voltage follower 120 in Fig. 1, which has a certain voltage value in proportion to a voltage difference between both ends of the electrode. Herein, voltage V2 is a fixed voltage approximate to a power source voltage of the stimulator, and therefore, {(K2 - K1) x V2} in Eq. (3) becomes a constant number and a voltage input to the voltage follower 120 becomes a value proportional to V2-V1. That is, V4 stored in the charge storage unit 110 may be appropriately adjusted by the resistors R1, R2, R3, and R4 such that it is within an operational input voltage range of the voltage follower 120.

While the invention has been shown and described with respect to the embodiments, the present invention is not limited thereto. It will be understood by those skilled in the art that various changes and modifications may be made without departing from the scope of the invention as defined in the following claims.

**Claims**

1. An apparatus for measuring interfacial impedance between a living body and a stimulating electrode, the apparatus comprising:

   a first electrode connected to one end of a cell within the living body;
   a second electrode connected to the other end of the cell within the living body, wherein a current is applied from a stimulator to the second electrode and is provided to the first electrode through the cell;
   a measurement unit configured to selectively extract a voltage induced to the first and second electrodes depending on the current applied to the first and second electrodes;
   a charge storage unit configured to store a relative potential corresponding to a voltage difference between the first and second electrodes;
   an A/D conversion unit configured to convert a signal corresponding to the relative potential into a digital signal; and
   an impedance calculation unit configured to calculate interfacial impedance of the first and second electrodes using the digital signal from the A/D conversion unit and the current applied to the second electrode.

2. The apparatus of claim 1, wherein the measurement unit comprises:

   a first switching unit connected to the first electrode and configured to perform a switching operation to connect the first electrode with the charge storage nit so that a voltage at the first electrode is stored in the charge storage unit; and
   a second switching unit connected to the second electrode and configured to perform a switching operation to connect the second electrode with the charge storage unit so that a voltage at the second electrode is stored in the charge storage unit,
   wherein when the first switching unit is turned on or turned off, the second switching unit is turned off or turned on.

3. The apparatus of claim 2, further comprising:

   a third switching unit connected in parallel to the charge storage unit and configured to perform a switching operation;
   a fourth switching unit connected in parallel to the charge storage unit and configured to perform a switching operation; and
   a voltage follower connected in parallel to the fourth switching unit, wherein the voltage follower provides an output signal corresponding to the relative potential stored in the charge storage unit to the A/D conversion unit as the relative potential is applied to one end thereof, and receives feedback of the output signal by the other end thereof.

4. The apparatus of claim 3, wherein the voltage at the first electrode is stored in the charge storage unit when the first and fourth switching units are turned on and the second and third switching units are turned off, and
   the relative potential between the first and second electrodes is applied to one end of the voltage follower when the second switching unit is turned on and the first, third, and fourth switching units are turned off.

5. The apparatus of claim 3, wherein a charge corresponding to the relative potential stored in the charge storage unit is discharged when the first and second switching units are turned off and the third and fourth switching units are turned on.

6. The apparatus of claim 1, wherein the measurement unit comprises:

   first and second resistors R1 and R2 connected in parallel to the first electrode;
   third and fourth resistors R3 and R4 connected in parallel to the second electrode;
   a fifth switching unit connected between the first and second resistors R1 and R2 and configured to perform a switching operation to connect the first electrode with the charge storage unit;
   a sixth switching unit connected in series to the second resistor R2 and configured to perform a switching operation to connect the second resistor R2 with the earth;
   a seventh switching unit connected between the third and fourth resistors R3 and R4 and configured to perform a switching operation to connect the second electrode with the charge storage unit; and
   an eighth switching unit connected in series to the fourth resistor R4 and configured to perform a switching operation to connect the fourth resistor R4 with the earth.

7. The apparatus of claim 6, wherein the fifth and sixth switching units are simultaneously turned on or turned off, and the seventh and eighth switching units are simultaneously turned on or turned off.

8. The apparatus of claim 6, further comprising:

a third switching unit connected in parallel to the charge storage unit and configured to perform an ON or OFF switching operation;
a fourth switching unit connected in parallel to the charge storage unit and configured to perform an ON or OFF switching operation; and
a voltage follower connected in parallel to the fourth switching unit, wherein the voltage follower provides an output signal corresponding to the relative potential stored in the charge storage unit to the A/D conversion unit as the relative potential is applied to one end thereof, and receive feedback of the output signal by the other end thereof.

9. The apparatus of claim 8, wherein the voltage at the first electrode is stored in the charge storage unit when the fourth, fifth, and sixth switching units are turned on and the third, seventh, and eighth switching units are turned off, and a relative potential between the first and second electrodes is applied to one end of the voltage follower when the seventh and eighth switching units are turned on and the third, fourth, fifth, and sixth switching units are turned off.

10. The apparatus of claim 9, wherein a charge corresponding to the relative potential stored in the charge storage unit is discharged when the fifth, sixth, seventh, and eighth switching units are turned off and the third and fourth switching units are turned on.

11. The apparatus of claim 1, wherein the impedance calculation unit calculates impedance by time at certain time intervals by using each digital signal from the A/D conversion unit and the current applied to the second electrode, and wherein the calculated impedance by time is assigned a unique index corresponding to measurement order.

12. The apparatus of claim 11, wherein the impedance calculation unit restores a voltage waveform taken to both ends of the first and second electrodes by using the impedance by time and the unique index to calculate a time constant in the interfacial impedance of the first and second electrodes.

13. The apparatus of claim 1, wherein the first and second electrodes are configured to be bipolar in which a pair of stimulating electrodes has the area with the same size.

14. The apparatus of claim 13, wherein the impedance calculation unit calculates a half value of impedance calculated by using the current applied to the second electrode and a digital signal from the A/D conversion unit, and wherein the calculated half value becomes the interfacial impedance of the first and second electrodes.

15. The apparatus of claim 1, wherein the first and second electrodes are configured to be monopolar in which the area of the second electrode is greater than that of the first electrode.

16. The apparatus of claim 15, wherein the impedance calculation unit calculates impedance calculated by using the current applied to the second electrode and a digital signal output from the A/D conversion unit, and wherein the calculated impedance becomes the interfacial impedance of the second electrode.

# FIG. 1

EP 2 564 772 A1

# FIG.2

# FIG.3

# FIG.4

# FIG.5

## INTERNATIONAL SEARCH REPORT

| International application No. |
| --- |
| **PCT/KR2010/002696** |

| A. | CLASSIFICATION OF SUBJECT MATTER |
| --- | --- |

*A61B 5/053(2006.01)i*

According to International Patent Classification (IPC) or to both national classification and IPC

| B. | FIELDS SEARCHED |
| --- | --- |

Minimum documentation searched (classification system followed by classification symbols)
A61B 5/053; A61B 5/04; A61B 5/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: bio, impedance, electrode

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| A | KR 10-0333166 B1 (BAOSPACE CO., LTD.) 18 April 2002<br>See abstract, page 4, line 30 - page 5, line 7, claims 1, 8, 11 and figures 1, 8 | 1-16 |
| A | KR 10-2004-0014852 A (PARK, WEON HEE) 18 February 2004<br>See abstract, claims 1-4, figure 5 | 1-16 |
| A | KR 10-2008-0012075 A (WELLTECHGLOBE PTY. LTD.) 11 February 2008<br>See abstract, claims 1-5, figures 4-5 | 1-16 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 JANUARY 2011 (17.01.2011) | **18 JANUARY 2011 (18.01.2011)** |
| Name and mailing address of the ISA/KR<br>Korean Intellectual Property Office<br>Government Complex-Daejeon, 139 Seonsa-ro, Daejeon 302-701, Republic of Korea<br>Facsimile No. 82-42-472-7140 | Authorized officer<br><br>Telephone No. |

Form PCT/ISA/210 (second sheet) (July 2009)

INTERNATIONAL SEARCH REPORT
Information on patent family members

International application No.

**PCT/KR2010/002696**

| Patent document cited in search report | Publication date | Patent family member | Publication date |
|---|---|---|---|
| KR 10-0333166 B1 | 18.04.2002 | US 6256532 B1 | 03.07.2001 |
| KR 10-2004-0014852 A | 18.02.2004 | NONE | |
| KR 10-2008-0012075 A | 11.02.2008 | NONE | |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4870341 A